(19) Europäisches Patentamt / European Patent Office / Office européen des brevets

(11) **EP 4 376 022 A1**

(12) **EUROPEAN PATENT APPLICATION**
published in accordance with Art. 153(4) EPC

(43) Date of publication:
**29.05.2024 Bulletin 2024/22**

(21) Application number: **22845343.7**

(22) Date of filing: **20.07.2022**

(51) International Patent Classification (IPC):
***G16H 70/20*** (2018.01)

(52) Cooperative Patent Classification (CPC):
**G16H 70/20**

(86) International application number:
**PCT/CN2022/106720**

(87) International publication number:
**WO 2023/001178 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.07.2021 US 202163224875 P**

(71) Applicant: **Kaohsiung Medical University Sanmin District Kaohsiung, Taiwan 80708 (CN)**

(72) Inventors:
- HUANG, Shu-Pin
  Kaohsiung, Taiwan 804051 (TW)
- LI, Chia-Yang
  Kaohsiung, Taiwan 807752 (TW)
- CHEN, Deng-Neng
  Pingtung City Pingtung County, Taiwan 900070 (TW)
- CHUNG, Wei-Ju
  Kaohsiung, Taiwan 807030 (TW)

(74) Representative: **Lang, Christian LangPatent Anwaltskanzlei IP Law Firm Ingolstädter Straße 5 80807 München (DE)**

(54) **CLINICAL THERAPEUTIC DRUG PREDICTION AND RECOMMENDATION SYSTEM AND METHOD FOR EVALUATING EFFICACY OF SECOND-GENERATION HORMONE DRUG IN TREATMENT OF PROSTATE CANCER**

(57)    A clinical therapeutic drug prediction and recommendation system (1) for evaluating the efficacy of a second-generation hormone drug in treatment of prostate cancer, comprising: an input device (10) used for receiving an ex vivo biological sample (40) and generating a physiological signal; a computer device (20) connected to the input device (10) and comprising a processor (201) used for receiving the physiological signal, said processor comprising an analysis module (2011) which compares at least the physiological signal with patient gene sets to which treatment by a first drug and a second drug are respectively applied, and then respectively obtains a first score and a second score corresponding to the first drug and the second drug by a computation method, the scores being further compared by a comparison module (2012), when the comparison result (20121) shows that the first score is greater than the second score, an indication (20131) of recommending the first drug to the individual being given, otherwise, an indication (20132) of recommending the second drug to the individual being given; and a display device, used for displaying the recommendation indication.

Figure 1A

EP 4 376 022 A1

**Description**

**FIELD OF THE INVENTION**

The present invention relates to a clinical therapeutic drug prediction and recommendation system, in particular a clinical therapeutic drug prediction and recommendation system for evaluating the efficacy of second-generation hormone drugs in the treatment of prostate cancer.

**BACKGROUND OF THE INVENTION**

According to the statistics of 2019, prostate cancer ranked sixth in Taiwan's cancer death ranking. It is well known that many prostate cancer patients are resistant to first-generation hormone therapeutic drugs, and because chronic inflammation causes induction, development and recurrence of prostate cancer, patients must subsequently receive second-generation hormone therapy.

The second-generation hormone therapeutic drugs are the first choice for patients' subsequent treatment. There are currently two types of second-generation hormone therapeutic drugs. One is a compound of androgen synthesis inhibitor Abiraterone, the other is a compound of androgen receptor blocker Enzalutamide, Apalutamide and Darolutamide. These two types of second-generation hormone therapies have therapeutic effects on treating metastatic castration-resistant prostate cancer. The two types of drugs both are health insurance drugs, but they cost a lot. Abiraterone costs about NT$90,000 per month, and Enzalutamide costs about NT$100,000 per month.

It may prolong the survival time of the patients when the patients randomly take one of the second-generation hormone drugs, but the efficacy of various second-generation hormone therapeutic drugs for different patients is largely different. If the efficacy is good, it can stably control cancer progression for several years. If the efficacy is poor, the drug resistance will develop after a few months of treatment. After the occurrence of drug resistance, the patients only are treated with follow-up chemotherapy, and their lives may come to an end in a short time. However, there is currently no technology, criterion or clinical literature to help doctors determine which patients are suitable for which drugs. Therefore, how to choose the appropriate second-generation hormone therapeutic drugs for the patients has become a major challenge in prostate cancer treatment.

**DETAILED DESCRIPTION OF THE INVENTION**

The present invention provides a clinical therapeutic drug prediction and recommendation system for evaluating the efficacy of second-generation hormone drugs in the treatment of prostate cancer, comprising: an input device for receiving ex vivo biological samples obtained from a subject, and generating at least one physiological signal (N) of the ex vivo biological samples; and a computer device connected to the input device, wherein the computer device comprises: a processor for receiving the at least one physiological signal, wherein the processor further comprises an analysis module for comparing the at least one physiological signal with a first-level gene set (X1) of patients suitable for a first drug treatment to obtain a comparison value ($N, X1$) and comparing the at least one physiological signal with a second-level gene set (X2) of the patients suitable for the first drug treatment to obtain a comparison value ($N, X2$), then obtaining a first score (S1) by using a calculation method, and comparing the at least one physiological signal with a first-level gene set (Y1) of patients suitable for a second drug treatment to obtain a comparison value ($N, Y1$) and comparing the at least one physiological signal with a second-level gene set (Y2) of the patients suitable for the second drug treatment to obtain a comparison value ($N, Y2$), then obtaining a second score (S2) by using the calculation method; a comparison module for comparing the first score (S1) and the second score (S2) to obtain a comparison result; and a recommendation module for receiving and analyzing the comparison result, wherein the recommendation module gives an indication to recommend the subject to take the first drug when the first score (S1) is higher than the second score (S2), or gives an indication to recommend the subject to take the second drug when the first score (S1) is lower than the second score (S2).

In the present invention, the computer device further comprises a display device for displaying the recommended indications.

In the present invention, the computer device further comprises a storage module for storing the at least one physiological signal, the comparison results and the recommended indications.

In a preferred embodiment of the present invention, the at least one physiological signal comprises genetic information of the ex vivo biological samples.

In a preferred embodiment of the present invention, the first drug is a compound of an androgen receptor blocker, and the second drug is a compound of an androgen synthesis inhibitor.

In the present invention, the calculation method comprises at least one algorithm.

In the present invention, the at least one algorithm comprises Euclidian Distance, Manhattan Distance, Hamming Distance, Cosine similarity algorithm or Jaccard Similarity (JS) coefficient.

In a preferred embodiment of the present invention, the algorithm is Jaccard Similarity coefficient.

In a preferred embodiment of the present invention, the Jaccard Similarity coefficient is used to calculate the comparison value of ($N, X1$), the comparison value of ($N, X2$), the comparison value of ($N, Y1$) and the comparison value of ($N, Y2$).

In the present invention, preferably, the calculation method further comprises a first weight value (W1) corresponding to the comparison value of ($N, X1$) or the comparison value of ($N, Y1$), and a second weight value (W2) corresponding to the comparison value of ($N, X2$) or the comparison value of ($N, Y2$).

In the present invention, preferably, the weight value ranges from 0 to 1.

The present invention further provides a clinical therapeutic drug prediction and recommendation method for evaluating the efficacy of second-generation hormone drugs in the treatment of prostate cancer, comprising: providing an input device for receiving ex vivo biological samples obtained from a subject and obtaining at least one physiological signal (N) from the ex vivo biological samples; providing a processor for receiving the at least one physiological signal; providing an analysis module for comparing the at least one physiological signal with a first-level gene set (X1) of patients suitable for a first drug treatment and comparing the at least one physiological signal with a second-level gene set (X2) of the patients suitable for the first drug treatment, then obtaining the first score (S1) by using a calculation method, and comparing the at least one physiological signal with a first-level gene set (Y1) of patients suitable for a second drug treatment and comparing the at least one of the physiological signals with a second-level gene set (Y2) of the patients suitable for the second drug treatment, then obtaining the second score (S2) by using the calculation method; providing a comparison module for comparing the first score (S1) and the second score (S2) to generate a comparison result; and providing a recommendation module for receiving the comparison result, wherein the recommendation module gives an indication to recommend the subject to take the first drug when the first score (S1) is higher than the second score (S2), or gives an indication to recommend the subject to take the second drug when the first score (S 1) is lower than the second score (S2).

## BRIEF DESCRIPTION OF THE DRAWINGS

Figures 1A to 1B show schematic diagrams of a clinical therapeutic drug prediction and recommendation system of the present invention. Figure 1A is a schematic diagram without a storage module. Figure 1B is a schematic diagram containing a storage module.

Figure 2 shows a flowchart of a clinical therapeutic drug prediction and recommendation method for evaluating the efficacy of the second-generation hormone drug in treatment of prostate cancer in the present invention.

Figures 3A to 3C show descriptions of the calculation method of the present invention. Figure 3A is a schematic diagram of an analysis algorithm based on the compound of androgen synthesis inhibitor Abiraterone. Figure 3B is a schematic diagram of a analysis algorithm based on the compound of androgen receptor blocker Enzalutamide. Figure 3C is a procedure for comparing the results of the patient responded to various drugs.

**Symbol description**

1: Clinical therapeutic drug prediction and recommendation system
10: Input device
20: Computer device
201: Processor
2011: Analysis module
2012: Comparison module
20121: Comparison result
2013: Recommendation module
20131: Indication of the use of the first drug
20132: Indication of the use of the second drug
30: Biological samples
40: Physiological signals
50: Storage module

**EXAMPLES**

The following embodiments are not intended to limit the scope of the present invention, but to make the above and other objects, features and advantages of the present invention more obvious.

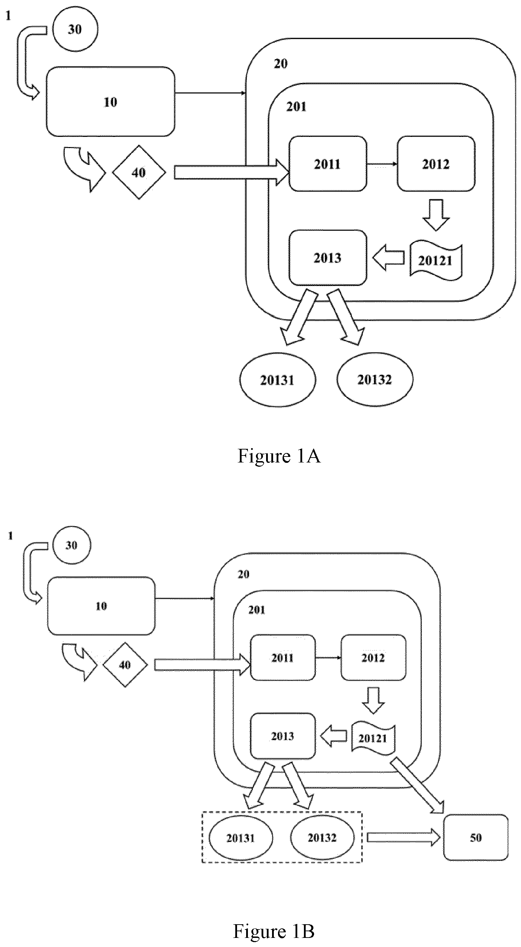

A clinical therapeutic drug prediction and recommendation system 1 of the present invention, as shown in Figure 1A, which was used to evaluate the efficacy of second-generation hormone drugs in the treatment of prostate cancer, comprising: an input device 10 for receiving ex vivo biological samples 40 obtained from a subject and generating at least one physiological signal (N) 50 of the ex vivo biological samples 40; and a computer device 20 connected to the input device 10, wherein the computer device 20 comprised: a processor 201 for receiving the at least one physiological signal 50, wherein the processor 201 further comprises an analysis module 2011 for comparing the at least one physiological signal 50 with a first-level gene set (X1) of patients suitable for a first drug treatment to obtain comparison value (N, X1) and comparing the at least one physiological signal with a second-level gene set (X2) of the patients suitable for the first drug treatment to obtain comparison value (N, X2), then obtaining the first score (S1) by using a calculation method, and comparing the at least one physiological signal with a first-level gene set (Y1) of patients suitable for a second drug treatment to obtain a comparison value (N, Y1) and comparing the at least one physiological signal with a second-level gene set (Y2) of the patients suitable for the second drug treatment to obtain comparison value (N, Y2), then obtaining a second score (S2) by using the calculation method; a comparison module 2012 for comparing the first score (S1) and the second score (S2) to obtain a comparison result 20121; and a recommendation module 2013 for receiving and analyzing the comparison result 20121, wherein the recommendation module gave an indication 20131 to recommend the subject to take the first drug when the first score (S1) was higher than the second score (S2), or gave an indication 20132 to recommend the subject to take the second drug when the first score (S1) was lower than the second score (S2).

Further, as shown in Figure 1B, the data or information could be transmitted to a storage module 60 of the computer device for storage, then the saved data or information could be used to perform algorithm calculations in the analysis module. After obtaining the data or information from the storage module, it was compared and calculated with the gene sets of patients suitable for different second-generation hormone drug treatments stored in the storage module or obtained by way of the network. Then, the results of the individual calculations were stored in the storage module, and displayed to the operator and the target patient by a display device (such as a screen, or monitor).

The present invention further comprised a display device. The display device could be a complete set of combination with the present invention or an independent external connection.

The input device of the present invention was a biochemical instrument well known to persons skilled in the art, including but not limited to: DNA sequencer, next-generation sequencer, biochemical serum analysis system, drug and special protein analyzer, electrolyte analyzer, glycated hemoglobin analyzer or osmotic pressure analyzer. In addition to receiving biological samples to be tested, the above biochemical instrument could be connected with the computer device by network, software and hardware integration for facilitating the performing analysis of the operator.

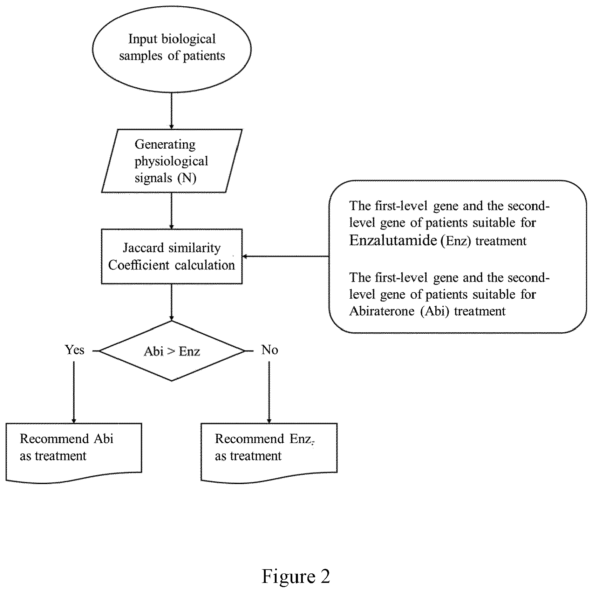

As shown in Figure 2, a flowchart of a clinical therapeutic drug prediction and recommended method of the present invention for evaluating the efficacy of second-generation hormone drugs in the treatment of prostate cancer was depicted. At first, obtaining biological samples from a target patient, wherein the biological samples could be blood, body fluids, hair, skin dander or other tissue fragments, and the biological sample was placed into the input device of the present invention to perform an input step, and the purpose was to be able to obtain the physiological signal (N) of the target patient, wherein the physiological signal was the gene of the target patient in a preferred embodiment, the genetic information was input into the processor of the computer device, and then the analysis step of physiological signal was performed by the analysis module of the processor, and the at least one physiological signal was compared with a first-level gene set (X1) of patients suitable for the first drug treatment, the at least one of the physiological signals was compared with a set second-level gene set (X2) of the patients suitable for the first drug treatment, and then calculated the first score (S1) by Jaccard similarity coefficient and similarity weighting, and compared the at least one physiological signal with a first-level gene set (Y1) of patients suitable for a second drug treatment, and compared the at least one physiological signal with a second-level gene set (Y2) of the patients suitable for the second drug treatment, and then calculated the second score (S2) by Jaccard similarity coefficient and similarity weighting. Then, it compared the first score (S 1) and the second score (S2) by the comparison module of the present invention, and the recommendation module of the present invention performed a recommendation step to give an indication to recommend the target patient to take the first drug when the first score (S 1) was higher than the second score (S2), or the recommendation module gave an indication to recommend the target patient to take the second drug when the first score (S1) was lower than the second score (S2).

In one embodiment, the first drug was a compound of an androgen receptor blocker (Enzalutamide; Enz), and the second drug was a compound of an androgen synthesis inhibitor (Abiraterone; Abi).

In one embodiment, the genetic collection of patients treated with different second-generation hormone drugs could be obtained from the network or previous analysis records.

Further, the data or information could be transmitted to the storage module of the computer device for storage, the saved data or information then could be used to perform algorithmic calculations by the analysis module. After the data or information were obtained from the storage module, it was compared and calculated with the gene set of patients suitable for different second-generation hormone drug treatments stored in the storage module or obtained by way of network, and then the results of the individual calculation were stored in the storage module and displayed to the operator and the target patient by the display device (such as a screen, or monitor).

The calculation formula of Jaccard similarity coefficient used in the present invention was shown in the following Equation (I). Generally, it represented a definition for comparing the similarity and difference between finite sample sets: given two sets A,B, and the Jaccard similarity coefficient was defined as the ratio of the size of the intersection of A and B to the size of the union of A and B.

$$J(A, B) = \frac{|A \cap B|}{|A \cup B|} = \frac{|A \cap B|}{|A| + |B| - |A \cap B|} \qquad \text{Equation (I).}$$

In the present invention, Jaccard similarity coefficient was used to calculate the comparison value of ($N, X1$), the comparison value of ($N, X2$), the comparison value of ($N, Y1$) and the comparison value of ($N, Y2$). Further, the weighting of the similarity was a first weight value (W1) corresponding to the comparison value of ($N, X1$) or the comparison value of ($N, Y1$), and a second weight value (W2) corresponding to the comparison value of ($N, X2$) or the comparison value of ($N, Y2$).

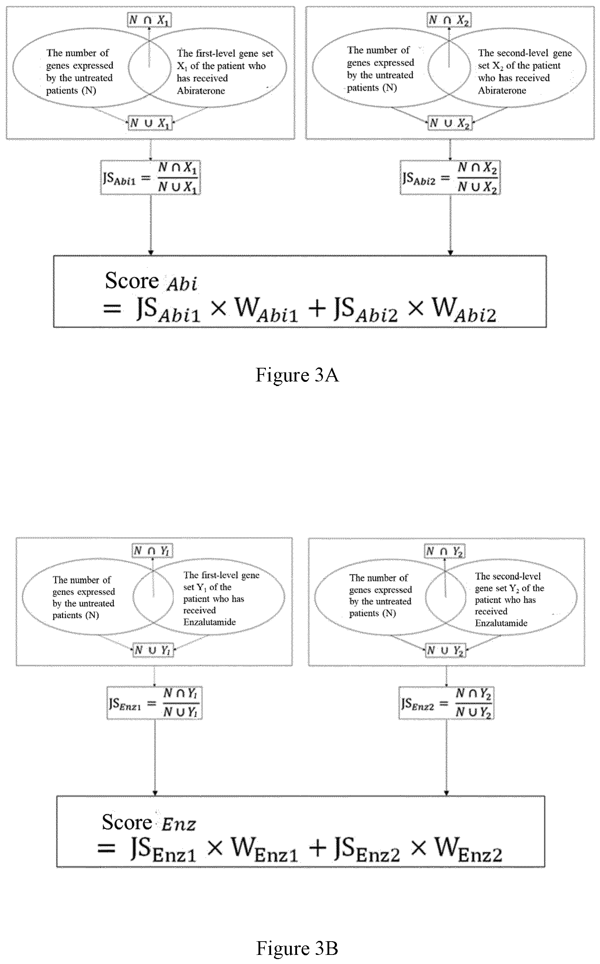

As shown in Figure 3A, an analysis algorithm based on the compound of androgen synthesis inhibitor Abiraterone (Abi) was taken as an example. The present invention performed multiple calculations by the algorithm for comparison and calculation. Each time, the number of genes expressed by the untreated patients (N) was provided and calculated with the first-level gene set (X1) of the patient who had received Abiraterone by Jaccard Similarity to obtain $JS_{Abi1}$; then the number of genes expressed by untreated patients (N) was provided and calculated with the secondary gene set (X2) of patients who has received Abiraterone by Jaccard Similarity to obtain $JS_{Abi2}$; and finally according to the following Equation (II), different weight values $W_{Abi1}$ and $W_{Abi2}$ were given respectively, and added together to obtain $Score_{Abi}$ which was used as the score of the patient responded to Abiraterone.

$$Score_{Abi} = JS_{Abi1} \times W_{Abi1} + JS_{Abi2} \times W_{Abi2} \qquad \text{Equation (II)}$$

As shown in Table 1, the first-level gene set (X1) regarding to Abiraterone for comparison contained 11 genes, and the second-level gene set (X2) regarding to Abiraterone for comparison contained 47 genes, wherein the genes of the first-level gene set (X1) were different from those of the second-level gene set (X2), and the weight values $W_{Abi1}$ and $W_{Abi2}$ were 1 and 0.8, respectively.

Table 1

| Gene sets | Number of genes | Gene name |
|---|---|---|
| X1 | 11 | NR4A1, ARHGEF10L, TNNT1, CPAMD8, CYP2S1, PLA2G7, CLEC10A, CD1C, ATF3, FCER1A, PLD4 |
| X2 | 47 | SHROOM1, OSM, CD83, STAB1, UCN, AC106782.1, ANKRD28, CD69, FCHO2, CCR3, CPA3, CCR1, IFNG, OXER1, GAS6, KCNC3, ZNF385A, ELANE, BPI, FAM109A, LCN2, AZU1, DEFA3, MZB1, PCSK6, KLF4, IFI27, SIGLEC1, LGALS2, LRP1, PLXNB2, GATA2, FOSB, ZNF703, HIST1H1C, NUCB2, JUN, C1orf54, CDC42EP1, LILRB4, TUBB2A, CD24, RHOB, ITGB3, TGFBI, AHSP, ZNF358 |

Figure 3B showed a schematic diagram of analysis algorithm that use of the compound of androgen receptor blocker Enzalutamide (Enz) as an example. The present invention performed multiple calculations by the algorithm for comparison and calculation as the same as Figure 3A. Taking this embodiment as an example, it performed two times calculations. Each time, the number of genes expressed by the untreated patients (N) were provided and calculated with the first-level gene set (Y1) of the patient who has received Enzalutamide by Jaccard Similarity to obtain $JS_{Enz1}$; then the number of genes expressed by untreated patients (N) were provided and calculated with the second-level gene set (Y2) of patients who have received Enzalutamide by Jaccard Similarity to obtain $JS_{Enz2}$; and finally according to the following equation (III), different weight values $W_{Enz1}$ and $W_{Enz2}$ were given respectively, and added together to obtain Score$_{Enz}$ which was used as the score of the patient responded to Enzalutamide.

$$Score_{Enz} = JS_{Enz1} \times W_{Enz1} + JS_{Enz2} \times W_{Enz2} \qquad \text{Equation (III)}$$

Regarding the algorithm calculation flow of Enzalutamide, as shown in Table 2, the first-level gene set (Y1) contained 5 genes, and the second-level gene set (Y2) contained 165 genes, wherein the gene sequences of the first-level gene set (Y1) were different from those of the second gene set (Y2), and the weight values $W_{Enz1}$ and $W_{Enz2}$ were 1 and 0.8, respectively.

Table 2

| Gene sets | Number of genes | Gene name |
|---|---|---|
| Y1 | 5 | TMF1, SLC4A1, ASH1L, ARMC2, STXBP5 |
| Y2 | 165 | SOD2, AQP9, FAM174A, SRSF11, ZBTB11, CDC42EP3, CDK17, NFIL3, GCA, HBG2, HEATR5B, MANF, TTC3, XAF1, APOBEC3A, STRN3, HBQ1, WSB1, TNFAIP3, ZDHHC17, MYSM1, ZMAT1, DDX3Y, CPEB4, ACSL1, FAM156A, ERBIN, WDR44, HBB, SLC39A10, AL132780.3, FPR2, TREM1, RFX2, ATRX, TNIK, PCMTD2, TMEM127, PLCG1, ZBTB38, PHC3, ANKRD28, CXCR2, DYNC1LI2, RNF103, EARS2, RNF19B, TLR8, HELZ2, ZNF518A, SLC38A2, MPZL1, CRLF1, DNAJC25-GNG10, RBL2, G0S2, CREBZF, LEMD3, TRAPPC10, MAP4K5, MGAM, PIK3R4, SCYL2, FAM198B, SDCBP, EIF5, PIGA, TAF1, KRR1, HIF1A, TLR4, GPD2, TCP11L2, PTP4A1, SREK1, ARID4B, KCTD12, TNFAIP8L2, TOB1, HBA2, BIRC3, PIKFYVE, UTRN, CDC14A, FAM76B, SENP7, GPR171, NKTR, GOLGA8N, NFKBIZ, GNS, PTGS2, ATXN7, ITGA6, PRF1, TNFRSF10C, RNPC3, KLF9, FAM91A1, PDK4, KIAA1109, ZNF548, HERC1, TRPM7, NAMPT, TNRC6A, YRDC, CXCR3, FCGR3B, PMAIP1, SOCS1, SLC25A36, GTF2I, LAMP2, NUAK2, COMMD3-BMI1, LPIN1, MLLT11, B4GALT5, SATB1, MYADM, FGL2, TC2N, CCL3L3, PROK2, RIC1, NPAT, GPATCH2L, CCNG2, GOLGA8A, STX11, MAN2A1, TFRC, UBE4A, UBE2D1, ZDHHC18, MYLIP, MARCKS, CPEB2, ARRDC3, RBM47, PAFAH1B2, MARCH1, SMC5, PTAFR, MORN2, ELL2, GOLGA8B, HBA1, CD83, LCN2, CD69, LTF, TUBB1, CHORDC1, 7-Mar, MCPH1, UGCG, TMEM68, HBM, OSBP2, PDZK1IP1, NRGN, SLPI, CLIC3 |

Wherein, the different-level gene sets of the two various drugs were further compared. It was accidentally found that the second-level gene set (X2) of abiraterone and the second-level gene set (Y2) of enzalutamide had partially duplicate genes.

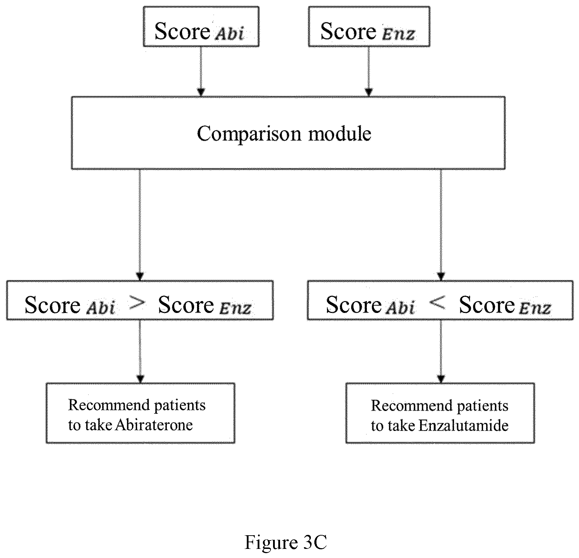

Finally, the results of the patients responded to various drugs were compared. As shown in Figure 3C, individual scores of the patient responded to Abiraterone (Abi) and the patient responded to Enzalutamide (Enz) were compared and determined by the comparison module of the computer device. If the score$_{Abi}$ > Score$_{Enz}$, the recommendation module recommended that the patient to choose Abiraterone as the preferred choice of the drug; otherwise. If the Score$_{Abi}$ < Score $_{Enz}$, the recommendation module recommended that the patient to choose Enzalutamide as the preferred choice of the drug.

In order to enable a person skilled in the art to which the present invention belongs can understand the method of making and using the technique, the present invention has been described and exemplified in sufficient detail, however, various variations, modifications or improvements should be regarded as equivalent to the spirit and scope of the present invention.

A person skilled in the art to which the present invention belongs can easily understand and achieve the object of the present invention, and obtain the previously mentioned results and advantages. The sources, weight values or gene sets of physiological signals used in the present invention are exemplary in nature and are not intended to limit the scope of the present invention. The spirit of the present invention covers those skilled in the art and modifications or other uses arising from the production or use of the art, and is limited by the scope of the claims.

**Claims**

1.　A clinical therapeutic drug prediction and recommendation system for evaluating the efficacy of second-generation hormone drugs in the treatment of prostate cancer, comprising:

an input device for receiving ex vivo biological samples obtained from a subject and generating at least one physiological signal (N) of the ex vivo biological samples; and
a computer device connected to the input device, wherein the computer device comprising:
a processor for receiving the at least one physiological signal, wherein the processor further comprises:

an analysis module for comparing the at least one physiological signal with a first-level gene set (X1) of patients suitable for a first drug treatment to obtain a comparison value (N, X1) and comparing the at least one physiological signal with a second-level gene set (X2) of the patients suitable for the first drug treatment to obtain a comparison value (N, X2), then obtaining a first score (S1) by using a calculation method, and comparing the at least one physiological signal with a first-level gene set (Y1) of patients suitable for a second drug treatment to obtain a comparison value (N, Y1) and comparing the at least one physiological signal with a second-level gene set (Y2) of the patients suitable for the second drug treatment to obtain a comparison value (N, Y2), then obtaining a second score (S2) by using the calculation method;
a comparison module connected to the analysis module for comparing the first score (S1) and the second score (S2) to obtain a comparison result; and
a recommendation module connected to the comparison module for receiving and analyzing the comparison result, wherein the recommendation module gives an indication to recommend the subject to take the first drug when the first score (S1) is higher than the second score (S2), or gives an indication to recommend the subject to take the second drug when the first score (S1) is lower than the second score (S2).

2. The system of claim 1, wherein the at least one physiological signal comprises genetic information of the ex vivo biological samples.

3. The system of claim 1, wherein the first drug is a compound of an androgen receptor blocker, and the second drug is a compound of an androgen synthesis inhibitor.

4. The system of claim 1, wherein the calculation method comprises at least one algorithm.

5. The system of claim 4, wherein the at least one algorithm comprises Euclidian Distance, Manhattan Distance, Hamming Distance, Cosine similarity algorithm or Jaccard Similarity coefficient.

6. The system of claim 5, wherein the algorithm is the Jaccard Similarity coefficient, and the Jaccard Similarity coefficient is used to calculate the comparison value of (N, X1), the comparison value of (N, X2), the comparison value of (N, Y1) and the comparison value of (N, Y2).

7. The system of claim 1, wherein the computer device further comprises a storage module for storing the at least one physiological signal, the comparison results and the recommendation indication.

8. The system of claim 1, wherein the calculation method further comprises a first weight value (W1) corresponding to the comparison value of (N, X1) or the comparison value of (N, Y1), and a second weight value (W2) corresponding to the comparison value of (N, X2) or the comparison value of (N, Y2).

9. The system of claim 8, wherein the weight value ranges from 0 to 1.

10. A clinical therapeutic drug prediction and recommendation method for evaluating the efficacy of second-generation hormone drugs in the treatment of prostate cancer, comprising:

providing an input step for receiving ex vivo biological samples obtained from a subject and obtaining at least one physiological signal (N) from the ex vivo biological samples and sending the at least one physiological signal to a processor;
providing an analysis step for comparing the at least one physiological signal with a first-level gene set (X1) of patients suitable for a first drug treatment and comparing the at least one physiological signal with a second-level gene set (X2) of the patients suitable for the first drug treatment, then obtaining a first score (S1) by using a calculation method, and comparing the at least one physiological signal with a first-level gene set (Y1) of patients suitable for a second drug treatment and comparing the at least one of the physiological signals with a second-level gene set (Y2) of the patients suitable for the second drug treatment, then obtaining a second score (S2) by using the calculation method;
providing a comparison step for comparing the first score (S1) and the second score (S2) to generate a comparison result; and

providing a recommendation step for receiving the comparison result, and giving an indication to recommend the subject to take the first drug when the first score (S1) is higher than the second score (S2) or giving an indication to recommend the subject to take the second drug when the first score (S1) is lower than the second score (S2).

Figure 1A

Figure 1B

Input biological
samples of patients

Generating
physiological
signals (N)

The first-level gene and the second-
level gene of patients suitable for
Enzalutamide (Enz) treatment

The first-level gene and the second-
level gene of patients suitable for
Abiraterone (Abi) treatment

Jaccard similarity
Coefficient calculation

Yes — Abi > Enz — No

Recommend Abi
as treatment

Recommend Enz.
as treatment

Figure 2

Figure 3A

Figure 3B

Figure 3C

## INTERNATIONAL SEARCH REPORT

| International application No. |
| --- |
| **PCT/CN2022/106720** |

**A. CLASSIFICATION OF SUBJECT MATTER**

G16H 70/20(2018.01)i

According to International Patent Classification (IPC) or to both national classification and IPC

**B. FIELDS SEARCHED**

Minimum documentation searched (classification system followed by classification symbols)

G16H

Documentation searched other than minimum documentation to the extent that such documents are included in the fields searched

Electronic data base consulted during the international search (name of data base and, where practicable, search terms used)

CNPAT, EPODOC, WPI, CNKI, ISI: 药物, 推荐, 前列腺癌, 基因, 治疗, 集合, 比对, 分数, 显示, 相似度, 加权, medicine, recommend, prostatic, cancer, gene, therapy, gather, compare, score, display, similarity, weighted

**C. DOCUMENTS CONSIDERED TO BE RELEVANT**

| Category* | Citation of document, with indication, where appropriate, of the relevant passages | Relevant to claim No. |
| --- | --- | --- |
| A | CN 111462921 A (SHANDONG UNIVERSITY) 28 July 2020 (2020-07-28) description, paragraphs [0005]-[0043], and figure 1 | 1-10 |
| A | CN 106778073 A (BEIJING GENE+ TECHNOLOGY CO., LTD.) 31 May 2017 (2017-05-31) entire document | 1-10 |
| A | CN 111785319 A (XIDIAN UNIVERSITY) 16 October 2020 (2020-10-16) entire document | 1-10 |
| A | US 6000828 A (POWER MED INC.) 14 December 1999 (1999-12-14) entire document | 1-10 |
| A | US 2017193157 A1 (MICROSOFT TECHNOLOGY LICENSING, LLC) 06 July 2017 (2017-07-06) entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 October 2022** | **21 October 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/106720**

| Patent document cited in search report | | | Publication date (day/month/year) | Patent family member(s) | | | Publication date (day/month/year) |
|---|---|---|---|---|---|---|---|
| CN | 111462921 | A | 28 July 2020 | None | | | |
| CN | 106778073 | A | 31 May 2017 | None | | | |
| CN | 111785319 | A | 16 October 2020 | None | | | |
| US | 6000828 | A | 14 December 1999 | WO | 9910821 | A1 | 04 March 1999 |
|  |  |  |  | AU | 9028798 | A | 16 March 1999 |
| US | 2017193157 | A1 | 06 July 2017 | WO | 2017116817 | A2 | 06 July 2017 |

Form PCT/ISA/210 (patent family annex) (January 2015)